(19) Europäisches Patentamt
European Patent Office
Office européen des brevets

(11) EP 3 100 734 A1

(12) **EUROPEAN PATENT APPLICATION**
published in accordance with Art. 153(4) EPC

(43) Date of publication:
**07.12.2016 Bulletin 2016/49**

(21) Application number: **14881170.6**

(22) Date of filing: **28.01.2014**

(51) Int Cl.:
***A61K 31/675*** *(2006.01)* ***A61P 35/00*** *(2006.01)*

(86) International application number:
**PCT/CN2014/000111**

(87) International publication number:
**WO 2015/113176 (06.08.2015 Gazette 2015/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Zhou, Wenqiang**
**Hunan 410205 (CN)**

(72) Inventor: **DENG, Jing**
**Changsha**
**Hunan 410205 (CN)**

(74) Representative: **Isarpatent**
**Patent- und Rechtsanwälte Behnisch Barth Charles**
**Hassa Peckmann & Partner mbB**
**Friedrichstrasse 31**
**80801 München (DE)**

(54) **USE OF CAMPTOTHECIN DERIVATIVE IN PREPARING PHARMACEUTICAL USED FOR TREATING MULTIPLE MYELOMA**

(57) The present invention relates to use of a camptothecin derivative for preparing a medicament for the treatment of multiple myeloma. The medicament is represented by Formula I and can be pharmaceutically acceptable salts thereof. This medicament shows advantages in treatment of multiple myeloma and avoids drug resistance problems for existing drugs.

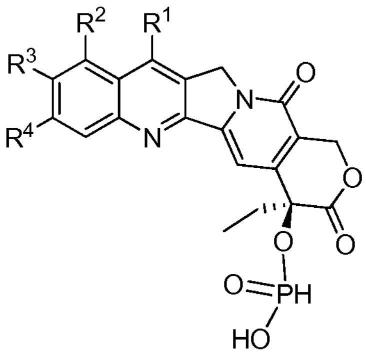

Formula I

EP 3 100 734 A1

## Description

### Field of the invention

The present invention relates to the field of pharmaceutics, particularly to the field of anticancer drugs, more particularly to pharmaceutical usage of compounds of small molecule drugs.

### Background of the invention

Multiple myeloma is a malignant clonal plasma cell disease, characterised in that clonal proliferation of malignant plasma cells in bone marrow of patients, which account for 10% of the hematopoietic neoplasm, and account for 1% of all malignant tumors. Currently the treatment of multiple myeloma is conducted by bone marrow transplantation in combination with chemotherapy (Review: The New England Journal of Medicine 2011, 364:1046-60; Clinical Lymphoma & Myeloma 2009, vol.9, No.4, 278-288). The lives of patients may be extended for several years with effective treatment (Blood 2008, 111:2521-2526). In the late twentieth century, standard chemotherapy drugs are DNA alkylating agents (such as, Melphalan and cyclophosphamide) and adrenocorticotropic hormones (such as Prednisone and dexamethasone). Although the chemotherapy regimens consisting of above chemotherapeutic agents are effective on some patients during initial treatment, these patients may inevitably develop resistance to these chemotherapy agents after a several courses of treatment, leading to disease recurrence and treatment failure. After that, thalidomide as "teratogenicy agent" as well as its derivative lenalidomide and Pomalidomide as immunomodulatory agents have been found to strengthen therapeutic effect of DNA alkylating agents and adrenocorticotropic hormone drugs, and were approved for clinical treatment around 2000. In 2003, a proteasome inhibitor Bortezomid as a chemotherapeutic agent with new mechanism has been approved for clinical treatment, that has some treatment effect for relapsed or refractory multiple myeloma with DNA alkylating agents and adrenocorticotropic hormone drug therapy. It can prolong life of the patients, but will also lose its effect because of development of resistance after several courses of treatment. Since the major chemotherapeutic agents used in clinical treatment for multiple myeloma may develop drug resistance after a period of time, the patients eventually will inevitably face the threat of cancer recurrence and death. Therefore, there is a pressing need to add more anti-cancer chemotherapeutic agents with non-cross resistance and having different pharmaceutical mechanism from prior art drugs, in order to provide a pharmaceutical basis for increasing effective medication regimen.

In clinical studies in recent years, topotecan was found to be useful for targeted therapeutic myeloma, and had some treatment effect for relapsed or refractory multiple myeloma with DNA alkylating agents and adrenocorticotropic hormone drug therapy, indicating the Prospects for applying topoisomerase type I inhibitor like camptothecins for the treatment of multiple myeloma (J Clin Oncol 1998, 16:589-592; Leukemia & Lymphoma 2004, vol.45, No.4, 755-759; Bone Marrow Transplantation 2011, 46: 510-515). Topotecan is a water-soluble derivative of natural occuring camptothecins, that is currently approved for the clinical treatment of a variety of other cancer chemotherapeutic agents (Review: Nature Review/Cancer, October 2006 Vol.6, pp789-802). Due to the impacts of attached chemical modification groups, topotecan has been identified as a substrate of cell resistance protein ABCG2/BCRP (Cancer Res 1999, 59: 5938-5946). ABCG2 is an ATP-binding transport membrane surface proteins and expressed in normal cells and tumor tissues in human. As a targeted drug efflux pumps, higher expression of ABCG2 may lead the tumor cells to develop multidrug resistance phenomenon. Because of the higher level of expression of ABCG2 protein in the resistant multiple myeloma cells, such highly expressed ABCG2/BCR proteins may pump topotecan outside these cancer cells, resulting in topotecan will lose therapeutic effect for killing the myeloma cells (Blood 2006, 108(12): 3881-3889; Biochem Pharmacol. 2012, 83(8): 1084-1103).

Therefore, it is necessary to develop a pharmaceutically acceptable novel camptothecins topoisomerase type-I inhibitor. On one hand, it may have good effect for killing multiple myeloma cancer cells. And on the other hand, it is not a substrate of cell resistance protein ABCG2/BCRP. The pharmaceutically acceptable novel camptothecins topoisomerase type-I inhibitor meeting the above requirements will be an important complement to chemotherapy for treatment of multiple myeloma.

### Summary of the invention

One object of the present invention is to provide uses of campthothcin derivative for treating multiple myeloma, the medicament has good effect on inhibition of bone marrow tumor growth. a pharmaceutical for treating cancer.

Another objection of the present invention is to provide uses of campthothcin derivative in preparing a medicament for the treatment of multiple myeloma, in order to solve a problem of multiple myeloma has resistant to existing drugs.

The compounds directed in the present invention is a CPT-phosphite of Formula I,

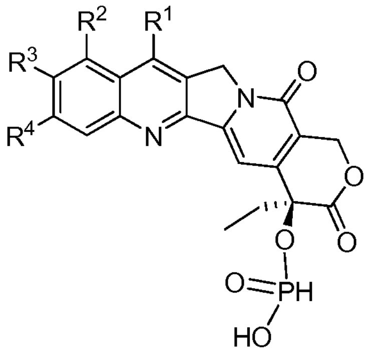

Formula I

or pharmaceutically acceptable salts thereof,

$R^1$ and $R^2$ independently represent hydrogen, hydroxy, nitro, cyano, halo, carboxy, optionally substituted amino, a silyl or siloxyl group containing C1-C6 alkyl group, mono-ring aryloxy, C1-C6 alkanoyl optionally substituted by hydroxy, nitro, cyano, halo or amino, C3-C6 cycloalkyl optionally substituted by hydroxy, nitro, cyano, halo or amino, C1-C6 alkoxy optionally substituted by hydroxy, nitro, cyano, halo or amino, or C1-C6 acyl optionally substituted by hydroxy, nitro, cyano, halo or amino; or $R^1$ and $R^2$ are connected via one to three other atoms forming a heterocycle;

$R^3$ and $R^4$ independently represent hydrogen, hydroxy, nitro, cyano, halo, carboxy, optionally substituted amino, a silyl or siloxyl group containing a C1-C6alkyl group, mono-ring aryloxy, C1-C6 alkanoyl optionally substituted by hydroxy, nitro, cyano, halo or amino, C3-C6 cycloalkyl optionally substituted by hydroxy, nitro, cyano, halo or amino, C1-C6 alkoxy optionally substituted by hydroxy, nitro, cyano, halo or amino, or C1-C6 acyl optionally substituted by hydroxy, nitro, cyano, halo or amino; or $R^3$ and $R^4$ independently represent oxygen and are connected via one or two canbon atoms forming a heterocycle.

Typically, cationic moiety of the pharmaceutically acceptable salts is selected from group consisting of $K^+$, $Na^+$, $Li^+$, $Mg^{2+}$, $Ca^{2+}$, $Zn^{2+}$, $Fe^{3+}$, and ammonium ion.

Preferably, the campthothcin derivative is derived from at least one selected from a group consisting of campthothcin, 10,11-ethylenedioxy CPT, 7-ethyl-10,11-ethylenedioxy CPT, 10,11-methylenedioxy CPT, 7-ethyl-10,11-methylenedioxy CPT, 7-chloro-10,11-methylenedioxy CPT, , 7-ethyl CPT,10-methyl-7-ethylCPT, 7-ethyl-10-chloro CPT, 7-ethyl-10-bromo CPT, 7-ethyl-10,11-difluoro CPT, 10-methyl-7-ethyl-11-fluoro CPT, 7-ethyl-10,11-dichloroCPT, Gimatecan, Karenitecan, Silatecan, 10-chloro CPT, 10-methylCPT, 10-ethyl CPT, 10-n-propyl CPT, 10-n-butyl CPT, 7-methoxyl CPT, 10-methyl-7-methoxyl CPT, 10-ethyl-7-methoxyl CPT, 10-n-propyl-7-methoxyl CPT, 10-n-butyl-7-methoxyl CPT. The most preferred is derived from campthothcin.

The compounds claimed in the present invention is a water-soluble CPT prodrug. At the physiological conditions, these compounds are cleaved by specific enzymes to release the bio-active camptothecin or camptothecin derivative with the 20(S)-hydroxyl group.

The compounds described above can be prepared pharmaceutical compositions for treating Multiple Myeloma in combination with other drugs having anti-cancer activity. The other drug having anti-cancer activity is selected from one or more of estrogen receptor modulator, androgen receptor modulator, retinoid receptor modulator, alkylating agents, tumor necrosis factor, tubulin inhibitor, topoisomerase inhibitors, anti-proliferative agents, acyltransferase Inhibitors HMG-CoA reductase inhibitor, protease inhibitor, and adrenal cortical hormone. Such other substance having anti-cancer activity is selected from one or more of, for example, tamoxifen, raloxifene, idoxifene, finasteride, nilutamide, flutamide, bicalutamide, bexarotene, vitamin A acid, 13-cis- retinoic acid, 9-cis-retinoic acid, Melphalan, ifosfamide, carboplatin, ranimustine, fotemustine, oxaliplatin, mitoxantrone, paclitaxel, topotecan, trimetrexate, fludarabine, capecitabine, thalidomide, lenalidomide, pomalidomide, prednisone, dexamethasone, bortezomid.

## Brief description of the drawings

Figure 1 shows concentration curve and CPT average plasma concentration after intravenous administration of 20 mg/kg (HM910).

Figure 2 shows growth curve graph of various groups of tumor in Example 3.

Figure 3 shows a picture of various groups of tumor block at the end of the experiment in Example.

## Detailed description of the invention

Unless otherwise specified, the terms used in context of the present invention are defined as in the following text. Other terms not defined in the following text have the definitions as commonly known in the field of the present invention.

The term "CPT prodrug" refers to the camptothecin derivative with the 20(S)-hydroxyl group protected by the biodegradable protecting group. At the physiological conditions, the biodegradable protecting group of the 20(S)-hydroxyl group is slowly cleaved by specific enzymes to generate the pharmaceutically active camptothecin.

The term "mammal" includes, but not limited to, primate, especially human; rodent includes mouse, rat, and hamster; domestic animal includes rabbit, horse, cow, dog and cat etc. In some embodiments, mammal refers to human.

The first aspect of the present invention relates to medical use of camptothecin derivatives represented by Formula I:

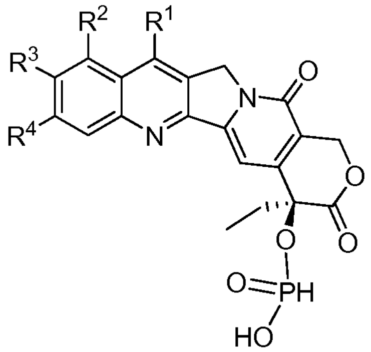

Formula I

wherein $R^1$ and $R^2$ independently represent hydrogen, hydroxy, nitro, cyano, halo, carboxy, optionally substituted amino, a silyl or siloxyl group containing C1-C6 alkyl group, mono-ring aryloxy, C1-C6 alkanoyl optionally substituted by hydroxy, nitro, cyano, halo or amino, C3-C6 cycloalkyl optionally substituted by hydroxy, nitro, cyano, halo or amino, C1-C6 alkoxy optionally substituted by hydroxy, nitro, cyano, halo or amino, or C1-C6 acyl optionally substituted by hydroxy, nitro, cyano, halo or amino; or $R^1$ and $R^2$ are connected via one to three other atoms forming a heterocycle;

$R^3$ and $R^4$ independently represent hydrogen, hydroxy, nitro, cyano, halo, carboxy, optionally substituted amino, a silyl or siloxyl group containing a C1-C6alkyl group, mono-ring aryloxy, C1-C6 alkanoyl optionally substituted by hydroxy, nitro, cyano, halo or amino, C3-C6 cycloalkyl optionally substituted by hydroxy, nitro, cyano, halo or amino, C1-C6 alkoxy optionally substituted by hydroxy, nitro, cyano, halo or amino, or C1-C6 acyl optionally substituted by hydroxy, nitro, cyano, halo or amino; or $R^3$ and $R^4$ independently represent oxygen and are connected via one or two canbon atoms forming a heterocycle.

Further, preferably, to allow CPT compounds of the present invention to exert bioactivity, $R^1$, $R^2$, $R^3$ and $R^4$ are selected from the groups of less steric hindrance to CPT, usually implemented by choosing those groups with smaller molecular weight, for example, controlling molecular weight of $R^1$, $R^2$, $R^3$ and $R^4$ under 100 respectively.

Perferably, the campthothcin derivatives of the present invention is in form of pharmaceutically acceptable salts thereof represented by Formula II.

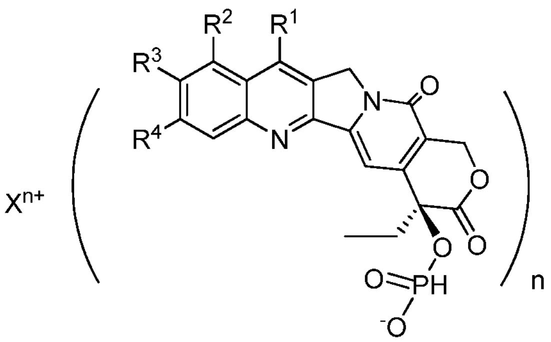

Formula II

Typically, the cationic moiety $X^{n+}$ is selected from $K^+$, $Na^+$, $Li^+$, $Mg^{2+}$, $Ca^{2+}$, $Zn^{2+}$, $Fe^{3+}$, or ammonium ion, wherein the ammonium ion preferably is $NH^{4+}$, and it does not rule out the ammonium ion can be derived from one of following bases: monomethylamine, dimethylamine, trimethylamine, monoethylamine, diethylamine, triethylamine, methylethylamine, dimethylethylamine, diisopropylamine, pyrrolidine, dihydro-isoindol, morpholine, N,N-diallyl amine, 4-methyl piperidine, ethanolamine, 5-bromo dihydro-isoindol, thiomorpholine, cis-2,6-dimethylmorpholine and ethylenediamine.

In addition to having good pharmaceutical activity, the above salthas desirable stability and better water solubility at the physiological conditions.

The inventors found that the campthothcin derivatives of the present invention and pharmaceutically acceptable salts thereof have good effects on treatment of multiple myeloma, and have different anti-resistance mechanisms with drugs for treating multiple myeloma that have been publicly reported.

The method for preparing the compounds related in the present intention has been disclosed in CN102850400A, the entirety of which is incorporated herein by reference.

In a more preferred aspect of the present invention, compounds of Formula II are derived from compounds of Formula IV as listed in Table 1 by introducing a phosphite moiety to the C-20 site. These compounds are not modified by hydroxy group or amino group at C9 site or C10 site.

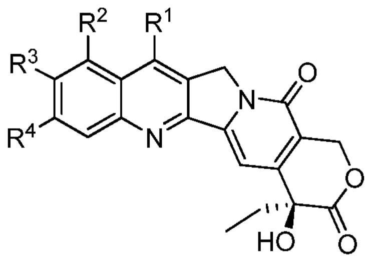

Formula IV

Table 1

| No. of Comps of Formula II | Name of Comps of Formula IV | Structural Formula of Comps of Formula IV | Structural Formula of Comps of Formula II | Molecular weight of Formula II |
|---|---|---|---|---|
| WQ1001 | Camptotheci n (CPT) | 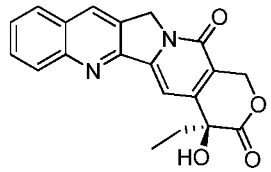 | 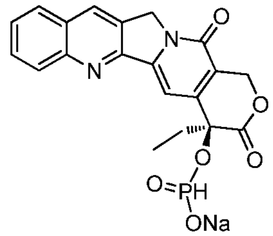 | 434.31 |
| WQ1102 | 10,11-ethyle nedioxy CPT | 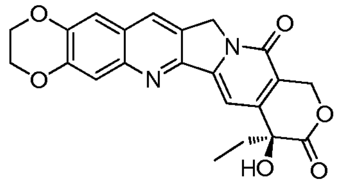 | 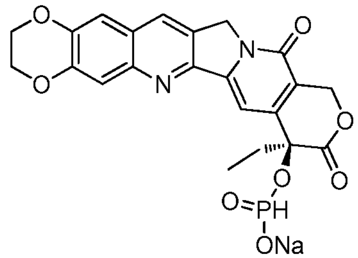 | 492.35 |
| WQ1103 | 7-ethyl-10,1 1-ethylenedi oxy CPT | 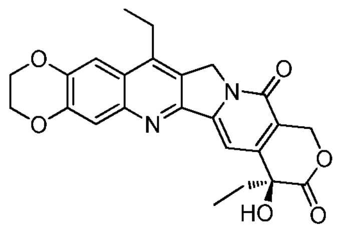 | 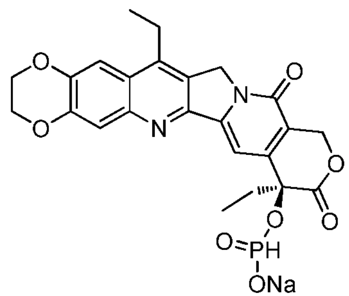 | 520.40 |

(continued)

| No. of Comps of Formula II | Name of Comps of Formula IV | Structural Formula of Comps of Formula IV | Structural Formula of Comps of Formula II | Molecular weight of Formula II |
|---|---|---|---|---|
| WQ1104 | 10,11-methyl enedioxy CPT | 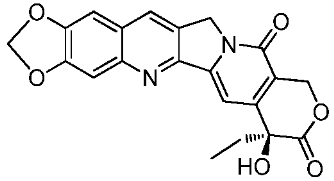 | 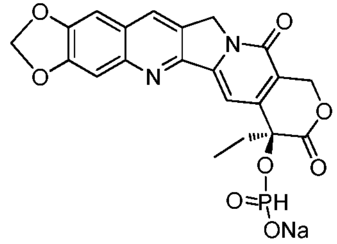 | 478.32 |
| WQ1105 | 7-ethyl-10,1 1-methylene dioxy CPT | 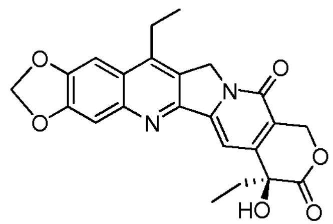 | 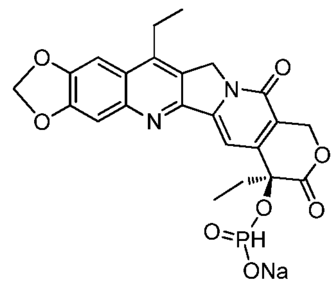 | 506.38 |
| WQ1106 | 7-chloro-10, 11-methylen edioxy CPT | 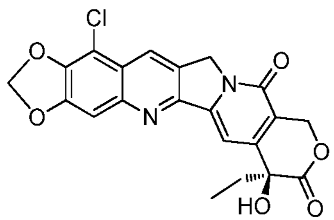 | 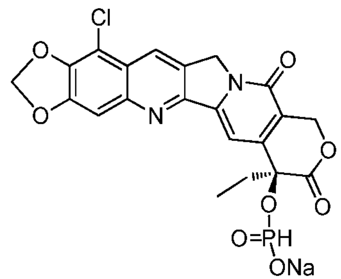 | 512.77 |

(continued)

| No. of Comps of Formula II | Name of Comps of Formula IV | Structural Formula of Comps of Formula IV | Structural Formula of Comps of Formula II | Molecular weight of Formula II |
|---|---|---|---|---|
| WQ1107 | 7-ethyl CPT | 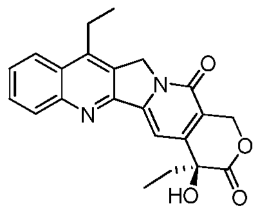 | 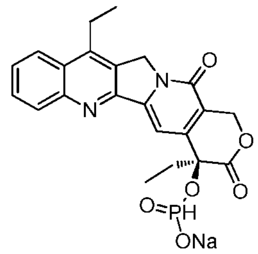 | 462.37 |
| WQ1108 | 10-methyl-7-ethyl CPT | 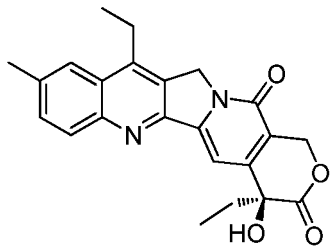 | 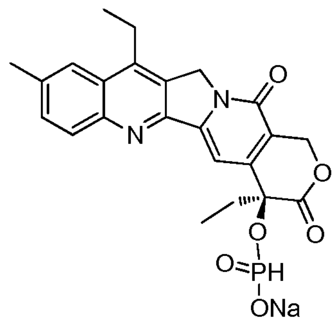 | 476.39 |
| WQ1109 | 7-ethyl-10-c hloro CPT | 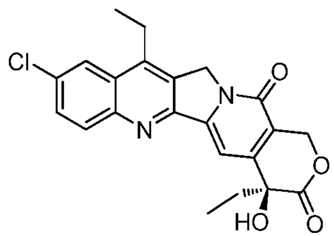 | 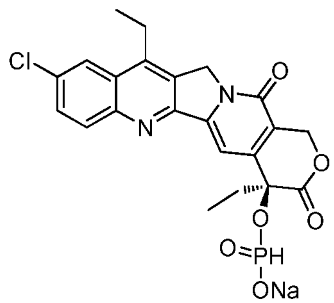 | 496.81 |

(continued)

| No. of Comps of Formula II | Name of Comps of Formula IV | Structural Formula of Comps of Formula IV | Structural Formula of Comps of Formula II | Molecular weight of Formula II |
|---|---|---|---|---|
| WQ1110 | 7-ethyl-10-br omo CPT | 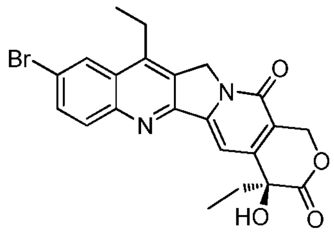 | 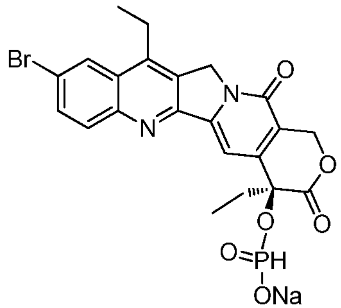 | 541.26 |
| WQ1111 | 7-ethyl-10,1 1-difluoro CPT | 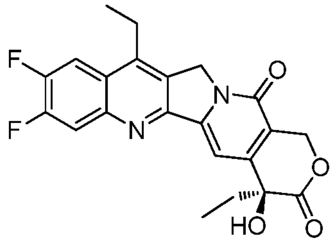 | 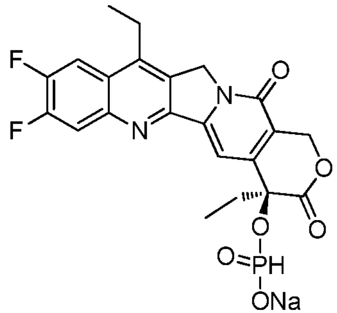 | 498.35 |
| WQ1112 | 10-methyl-7-ethyl-11-fluo ro CPT | 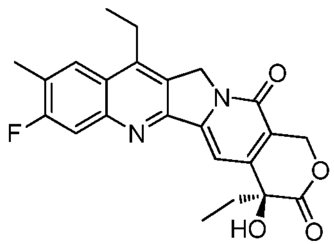 | 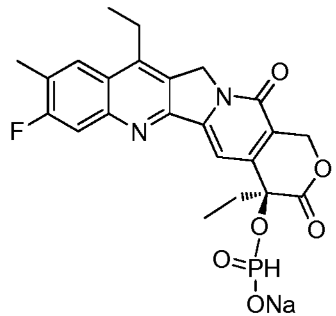 | 494.38 |

(continued)

| No. of Comps of Formula II | Name of Comps of Formula IV | Structural Formula of Comps of Formula IV | Structural Formula of Comps of Formula II | Molecular weight of Formula II |
|---|---|---|---|---|
| WQ1113 | 7-ethyl-10,1 1-dichloro CPT | 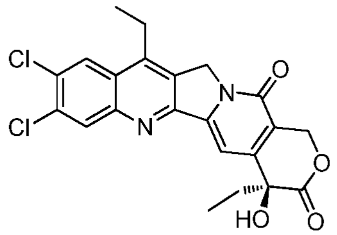 | 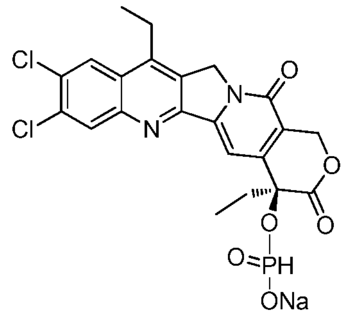 | 531.26 |
| WQ1114 | Gimatecan | 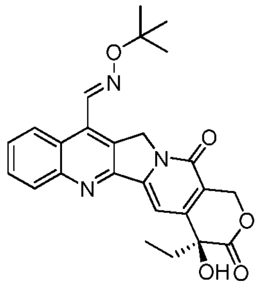 | 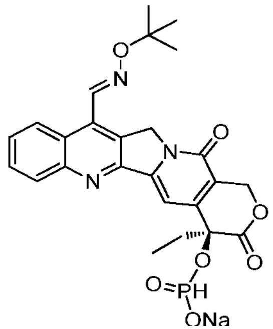 | 533.45 |
| WQ1115 | Karenitecan | 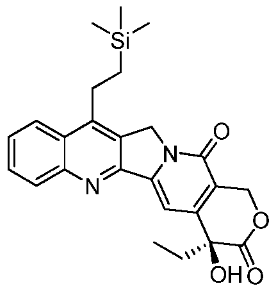 | 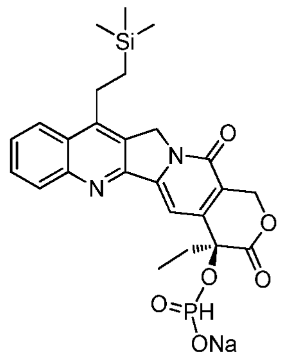 | 534.55 |

(continued)

| No. of Comps of Formula II | Name of Comps of Formula IV | Structural Formula of Comps of Formula IV | Structural Formula of Comps of Formula II | Molecular weight of Formula II |
|---|---|---|---|---|
| WQ1116 | Silatecan | 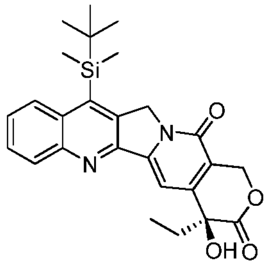 | 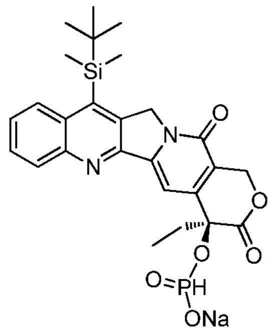 | 548.58 |
| WQ1117 | 10-chloro CPT | 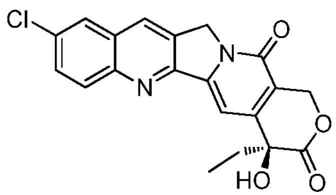 | 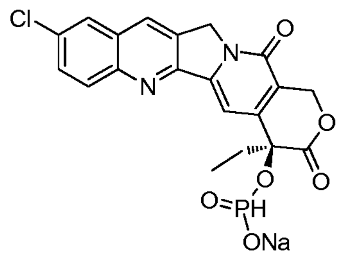 | 468.76 |
| WQ1118 | 10-methyl CPT | 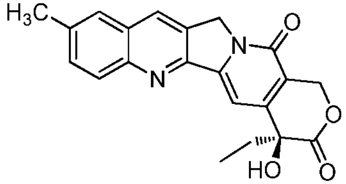 | 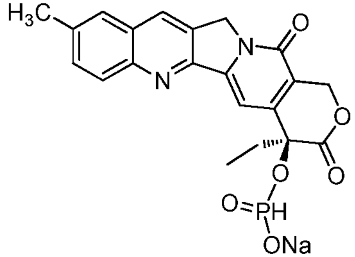 | 448.34 |

(continued)

| No. of Comps of Formula II | Name of Comps of Formula IV | Structural Formula of Comps of Formula IV | Structural Formula of Comps of Formula II | Molecular weight of Formula II |
|---|---|---|---|---|
| WQ1119 | 10-ethyl CPT | 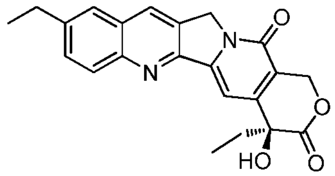 | 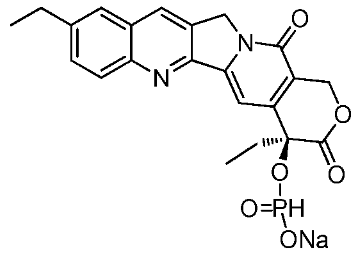 | 462.37 |
| WQ1120 | 10-n-propyl CPT | 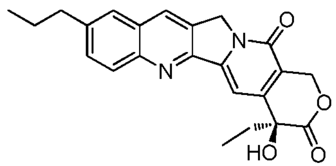 | 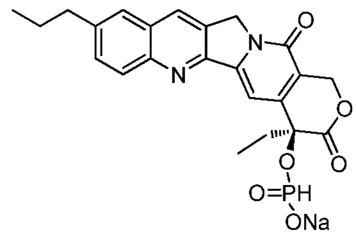 | 476.39 |
| WQ1121 | 10-n-butyl CPT | 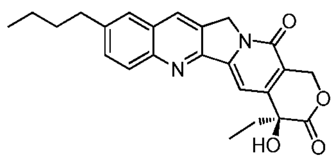 | 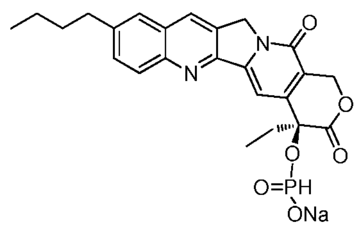 | 490.42 |
| WQ1122 | 7-methoxyl CPT | 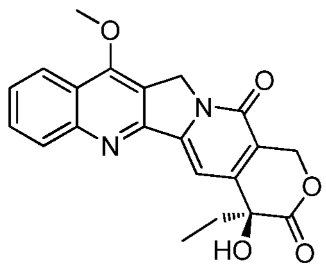 | 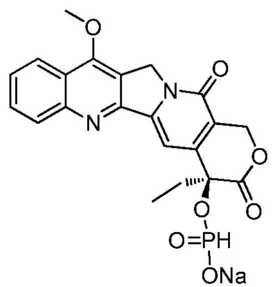 | 464.34 |

(continued)

| No. of Comps of Formula II | Name of Comps of Formula IV | Structural Formula of Comps of Formula IV | Structural Formula of Comps of Formula II | Molecular weight of Formula II |
|---|---|---|---|---|
| WQ1123 | 10-methyl-7-methoxyl CPT | 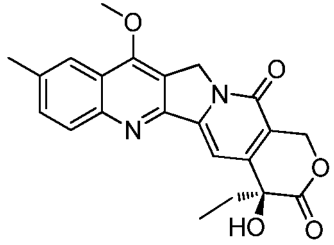 | 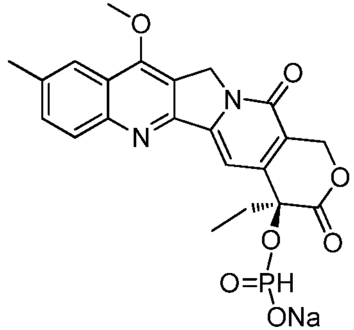 | 478.37 |
| WQ1124 | 10-ethyl-7-m ethoxyl CPT | 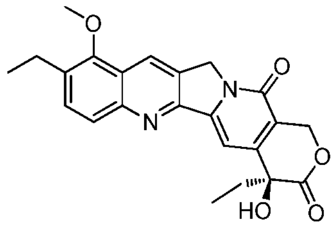 | 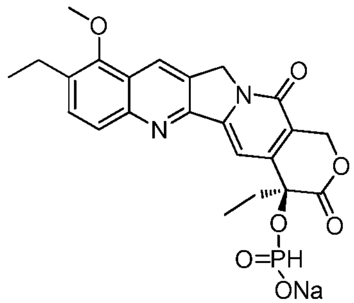 | 492.39 |
| WQ1125 | 10-n-propyl -7-methoxyl CPT | 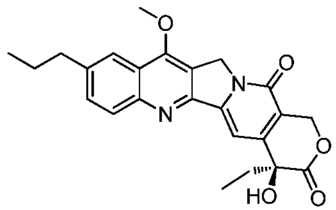 | 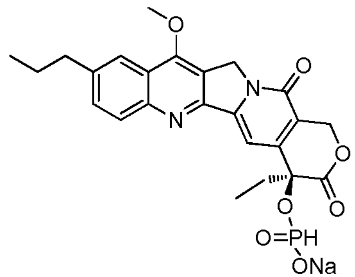 | 506.42 |

(continued)

| No. of Comps of Formula II | Name of Comps of Formula IV | Structural Formula of Comps of Formula IV | Structural Formula of Comps of Formula II | Molecular weight of Formula II |
|---|---|---|---|---|
| WQ1126 | 10-n-butyl -7-methoxyl CPT | 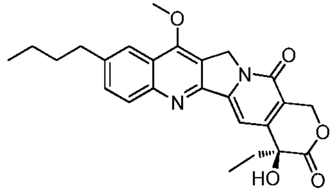 | 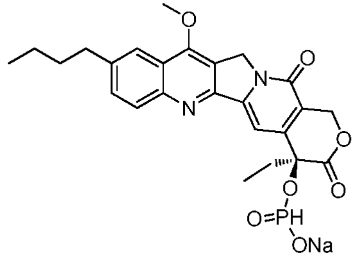 | 520.45 |

In administering the compound of the present invention to patients in need of such treatment, an effective amount of the compound or formulation containing one or more compounds of the present invention is administered to the patient. As used herein, the term "effective amount" is intended to mean the amount that the compound of the present invention will result in a desirable effect. For example, for treatment on cancer/malignant tumor, the "effective amount" refers to the amount which will inhibit, or retard the development of cancer, or kill cancer or malignant cells, e.g. regressing and/or palliating symptoms of malignant tumors, e.g., reducing the volume or size of such tumors or eliminating the tumor entirely. The effective amount (dosage) of compounds of the present invention is preferably between 0.1 to 100 mg of one compound of the present invention per kg of body weight. More preferably, the effective amount (dosage) is between 1 to 50 mg of one or more of compound(s) of the present invention per kg of body weight. If necessary or feasible as deemed by a doctor or veterinarian, the effective amount may be beyond the scope mentioned above. When the compound of the present invention is administered by way of its pharmaceutically acceptable salt, solvate or hydrate, the effective amount refers to the amount of free compound.

The compounds and pharmaceutical compositions according to the present invention can be administered by any route, for example, orally, nasally, parenterally, intravenously, intradermally, subcutaneously, or topically, in liquid or solid form.

For the purposes of parenteral therapeutic administration, the active ingredient may be incorporated into a solution or suspension. The solutions and suspensions may also include the following components: a sterile diluent for injection such as water for injection; suspensions of liposomal particles whereby the particles contain stable, active drug within the core of the particle in a pH controlled and protected environment; suspensions of liposomal particles, whose active drug is attached to the outside of the particle or either of the bilayers of the particle; saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediamine tetraacetic acid; buffers such as acetates, citrates and agents for adjusting tonicity such as sodium chloride or dextrose. The parenteral preparation can be enclosed in ampoules, disposable syringes or multiple dose vials made of glass or plastic.

Oral compositions generally include an inert diluent or an edible carrier. They may be enclosed in gelatin capsules or compressed into tablets. For the purpose of oral therapeutic administration, the aforesaid compounds may be prepared in the form of tablets, pills, capsules, troches, elixirs, suspensions, syrups, wafers, chewing gums and the like. The tablets, pills, capsules and the like may contain the following ingredients: a binder such as microcrystalline cellulose, gum tragacanth or gelatin; an excipient such as starch or lactose, a disintegrating agent such as alginic acid, Primogel, corn starch and the like; a lubricant such as magnesium stearate or Sterotes; a glidant such a colloidal silicon dioxide; and a sweetening agent such as sucrose or saccharin; or flavoring agent such as peppermint, methyl salicylate, or orange flavoring may be added. When the dosage unit is in the form of a capsule, it may contain, in addition to material of the above type, a liquid carrier such as a fatty oil. Other dosage-unit forms may contain other various materials which modify the physical form of the dosage unit, for example, coatings. Thus tablets or pills, for example, may be coated with sugar, shellac, or other enteric coating agents. A syrup may contain, besides the active compounds, sucrose as a sweetening agent, and a preservative, a dye and a coloring agent and a flavor. Materials used in preparing these compositions should be pharmaceutically or veterinarally pure and non-toxic in the amount used.

The compound according to the present invention can be used in combination with one or more other anti-cancer drugs. The other anti-cancer drugs in the context include: 1) estrogen receptor modulator, e.g., tamoxifen, raloxifene, idoxifene; 2) androgen receptor modulator, e.g., finasteride, nilutamide, flutamide, bicalutamide; 3) retinoid receptor modulator, e.g., bexarotene, vitamin A acid, 13-cis- retinoic acid, 9-cis-retinoic acid; 4) cytotoxic substances, including alkylating agents, tumor necrosis factor, tubulin inhibitor, topoisomerase inhibitors, e.g., ifosfamide, carboplatin, ranimustine, fotemustine, oxaliplatin, mitoxantrone, paclitaxel, and topotecan; 5) anti-proliferative agents, e.g. trimetrexate, fludarabine, and capecitabine; 6) acyltransferase Inhibitors; 7) HMG-CoA reductase inhibitor; 8) HIV protease inhibitor, and 9) reverse transcriptase inhibitor, etc.

A compound of Formula I or Formula II according to the persent invention is transported via the blood circulation system after entering the body, which is depredated into corresponding compound of formula IV through enzymatic reactions.

Preferably, compound of Formula I or Formula II according to the persent invention is derived from camptothecin or compounds represented by Formula IV as listed in Table 1 without modification of hydroxy group or amino group at C9 site or C10 site (e.g. campthothcin, 10,11-ethylenedioxy CPT, 7-ethyl-10,11-ethylenedioxy CPT, 10,11-methylenedioxy CPT,7-ethyl-10,11-methylenedioxy CPT,7-chloro-10,11-methylenedioxy CPT, 7-ethyl CPT, 10-methyl-7-ethyl CPT, 7-ethyl-10-chloro CPT, 7-ethyl-10-bromo CPT, 7-ethyl-10,11-difluoro CPT, 10-methyl-7-ethyl-11-fluoro CPT, 7-ethyl-10,11-dichloroCPT, Gimatecan, Karenitecan, Silatecan, 10-chloro CPT, 10-methylCPT, 10-ethyl CPT,10-n-propyl CPT, 10-n-butylCPT, 7-methoxyl CPT, 10-methyl-7-methoxyl CPT, 10-ethyl-7-methoxyl CPT, 10-n-propyl-7-methoxyl CPT, 10-n-butyl-7-methoxyl CPT), whose efficiency for being selectively pumped outside the cells after entering the cell by proteins BCRP/ABCG2 on surface membrane is much lower than topotecan.

Camptothecin or compounds of Formula IV as listed in Table 1 itself has poor water solubility, and is not suitable to be administered directly. By substitution of hydroxyl group with a phosphite at C-20 site, the resulted corresponding compounds represented by Formula II of the present invention have good water solubility.

Example 1: Changes in plasma concentration of WQ1001 and its metabolites CPT in rats after intravenous injection of WQ1001

Intravenous administration in rats: six healthy SD rats, weighing 180 ~ 220 g, three males and three females. Fasting for 12 h before dosing, free access to water. 20 mg/kg of HM 910 was administrated by intravenous bolus injection though caudal vein of the rats, with dose-volume volume of 1.0 mL/kg. Blooding 0.25 mL from the animal through the jugular vein, anti-clotting with heparin sodium, adding 0.2% formic acid within the anticoagulant. Blooding at following time points: 5 min, 15 min, 30 min, 45 min, 1 h, 1.5 h, 2 h, 2.5 h, 3 h, 4 h before and after administration. Blood samples were placed on ice after blooding and the plasma was centrifugal separated within 30 minutes (centrifugation conditions: 8000 r/min, 6 minutes, room temperature). The plasma collected was stored at -80°C for analysis. Table 2 and Figure 1 show drug concentration in the rats at different times after administration. That can be seen, the tested drug was degraded and the releases camptothecin after injecting into the rats.

Table 2: Drug concentration (ng/ml) in the rats at different times after intravenous administration

| Rats | **1** | **2** | **3** | **4** | **5** | **6** | **Mean** | **SD** |
|---|---|---|---|---|---|---|---|---|
| Time **(hr)** | HM 910 Plasma **(ng/mL)** | | | | | | | |
| **0** | BLQ | BLQ | BLQ | BLQ | BLQ | BLQ | **NA** | **NA** |
| **0.083** | 56652 | 31029 | 37348 | 56722 | 45165 | 21141 | **41343** | **14254** |
| **0.25** | 13690 | 9484 | 7271 | 16294 | 6761 | 5639 | **9856** | **4250** |
| **0.5** | 2530 | 1425 | 1633 | 4582 | 1536 | 1628 | **2223** | **1222** |
| **0.75** | 919 | 574 | 539 | 1578 | 617 | 607 | **806** | **402.2** |
| **1** | 499 | 259 | 240 | 668 | 240 | 256 | **360.2** | **181.0** |
| **1.5** | 97.2 | 78.0 | 45.2 | 162 | 59.0 | 96.4 | **89.6** | **40.8** |
| **2** | 18.3 | 25.7 | 51.5 | 39.5 | 20.8 | 53.7 | **34.9** | **15.5** |
| **2.5** | 28.2 | BLQ | BLQ | 17.2 | 18.4 | 11.0 | **18.7** | **NA** |
| **3** | BLQ | BLQ | BLQ | BLQ | BLQ | BLQ | **NA** | **NA** |
| **4** | BLQ | BLQ | BLQ | BLQ | BLQ | BLQ | **NA** | **NA** |
| Time **(hr)** | CPT Plasma Concentration **(ng/mL)** | | | | | | | |
| **0** | BLQ | BLQ | BLQ | BLQ | BLQ | BLQ | **NA** | **NA** |
| **0.083** | 6920 | 6450 | 6669 | 4092 | 6451 | 4697 | **5880** | **1179** |
| **0.25** | 12649 | 7935 | 7656 | 7878 | 7194 | 7812 | **8521** | **2040** |
| **0.5** | 11096 | 4385 | 5455 | 10296 | 4998 | 10090 | **7720** | **3076** |
| **0.75** | 7123 | 2397 | 2441 | 6595 | 2490 | 3941 | **4165** | **2173** |
| **1** | 4428 | 1100 | 1160 | 4442 | 1149 | 1733 | **2335** | **1643** |
| **1.5** | 945 | 279 | 217 | 1234 | 327 | 531 | **589** | **412** |
| **2** | 67.1 | 90.9 | 349 | 339 | 126 | 206 | **196** | **124** |
| **2.5** | 158 | 41.1 | 20.8 | 129 | 68.0 | 60.8 | **79.6** | **53** |
| **3** | 72.8 | 25.2 | 18.6 | 56.4 | 30.9 | 40.4 | **40.7** | **20** |
| **4** | 25.2 | 11.1 | 8.0 | 21.2 | 13.1 | 14.4 | **15.5** | **6.5** |

Example 2: Anti-drug resistance test

Logarithmic growth phase S1 and S1-M1-80 (characteristic expression of ABCG2/BCRP) were prepared as cell suspension of 2.5x104 cells/ml and 6x104 cells/ml, and were seeded in into 96-well plates with 0.19 ml/well. After incubating in cell incubator for 24 hours at 37°C, 5% $CO_2$, sample solutions in various concentrations were added at 0.01 ml/well. Stroke-physiological saline solution is used as control well. Each treatment is conducted in 3 parallel wells. The drugs were added and incubated additional 72 h hours. And 5 mg / ml of MTT is added at 0.02 ml/well before four hours of the end the incubation. The culture liquid is removed at the end of incubation. Each well was added 0.1 ml of DMSO and OD values were measured at wavelength of 540 nm and reference wavelength of 655 nm. The experiment

was repeated three times. Equation 1 is used to calculate cell growth inhibition rate (IR); and Equation 2 is used to calculate resistance index. Bliss method is used to calculate the 50% inhibitory concentration (IC50). The resistance indices for various drugs were shown in Table 3.

Table 3: Resistance indices of typical compounds

| Compounds | Active substances metabolized in vivo | Resistance index |
|---|---|---|
| WQ1001 | CPT | 6 |
| WQ1102 | 10,11-ethylenedioxyCPT | 15 |
| WQ1105 | 7-ethyl-10,11-methylene dioxy CPT | 20 |
| WQ1108 | 10-methyl-7-ethyl CPT | 38 |
| WQ1111 | 7-ethyl-10,11-difluoro CPT | 7 |
| WQ1114 | Gimatecan | 19 |
| WQ1115 | Karenitecan | 25 |
| WQ1116 | Silatecan | 13 |
| WQ1117 | 10-chloro CPT | 9 |
| WQ1118 | 10-methyl CPT | 67 |
| WQ1122 | 7-methoxyl CPT | 5 |
| Topotecan (TPT) | Topotecan | 210 |
| SN38 | SN38 | 290 |

It can be seen that from above resistance index that, in Anti-drug resistance test, the camptothecin derivatives of the present invention have the resistance index more than 10x lower than existing camptothecin derivatives (such as topotecan) It can be predicted that, when the compounds of the present invention were prepared to medicaments and used in therapeutic applications of bone marrow cancer after clinical testing in the future, they will have better anti-drug resistance than topotecan.

Example 3: Tumor inhibitory effect of WQ1001 on multiple myeloma cells NCI-H929 xenografts in nude mice

Multiple myeloma cells were routinely passaged in vitro, inoculated in 40 6-7-week-old BALB/c nude mouse, each mouse was subcutaneously injected human multiple myeloma cells NCI-H929 at its right axillary. Weighed after two days. The mice were randomly divided into five groups, eight in each group. Each group was dosed as follows:

i. physiological saline control group

ii. Topotecan positive control group, 2 mg/kg, once every 4 days, intraperitoneal injection

iii. WQ1001, high-dose group, 35 mg/kg, once every 4 days, intraperitoneal injection

iv. WQ1001, middle dose group, 25 mg/kg, once every 4 days, intraperitoneal injectionv. v. WQ1001, low dose group, 18 mg/kg, once every 4 days, intraperitoneal injection

Body weight(BW) as well as long diameter and short diameter of tumors were measured every two days. Tumor volume was calculated according to the Equation 1. On the 11th day after inoculation, the mice were weighed, long diameter and short diameter of tumors were measured, and relative tumor volume (RTV) was calculated according to Equation 2. Mice were sacrificed by cervical dislocation and the tumor mass was stripped and weighed. Inhibition rate (IR) was calculated according to the Equation 3. RTV and tumor weight (TW) for each group were analyzed through T-test. Test results were provided in Table 4 and Figure 3.

$$\text{Tumor Weight} = \text{long diameter} \times \text{short diameter}^2 \times \frac{1}{2} \quad \text{(Equation 1)}$$

$$RTV = \frac{\text{Tumor volume at the end of the test}}{\text{Tumor volume at the begaining of the test}} \quad \text{(Equation 2)}$$

$$IR = \frac{\text{mean RTV (or TW)}_{\text{control group}} - \text{mean RTV (or TW)}_{\text{test group}}}{\text{mean RTV (or TW) of control group}} \times 100\% \quad \text{(Equation 1)}$$

Table 4: Inhibitory effect of WQ1001 (HM910) and the like on NCI-H929 xenografts in nude mice

| Groups | Dose ml/kg | No. of Animlals initial | No. of Animlals end | Bw ($\overline{X}$ ±SD, g) initial | Bw ($\overline{X}$ ±SD, g) End | T V ($\overline{X}$ ±SD, $mm^3$) initial | T V ($\overline{X}$ ±SD, $mm^3$) End | RTV ($\overline{X}$ ±SD) | IR By RTV % | IR By RTV $P$ | TW ($\overline{X}$ ±SD, g) | IR By TW % | IR By TW $P$ |
|---|---|---|---|---|---|---|---|---|---|---|---|---|---|
| NS | 10 | 8 | 8 | 17.4 ±0.7 | 20.6 ±1.4 | 49.0 ±12.0 | 1972.6 ±1004.0 | 40.3±18.5 | | | 1.49±0.89 | | |
| Topotecan | 2 | 8 | 8 | 17.2 ±1.0 | 19.2 ±1.3 | 45.9 ±6.0 | 1123.2 ±608.9 | 25.3±14.8 | 37.3 | 0.0953 | 0.71±0.44 | 52.0 | 0.0451 |
| HM910 h-dose | 35 | 8 | 8 | 17.3 ±0.7 | 18.8 ±0.6 | 46.9 ±8.8 | 272.3± 483.1 | 5.61±9.74 | 86.1 | 0.0004 | 0.18±0.36 | 88.2 | 0.0018 |
| HM910 m-dose | 25g | 8 | 8 | 17.3 ±0.7 | 18.5 ±0.7 | 48.2 ±7.8 | 480.1± 570.6 | 9.80±11.6 | 75.7 | 0.0015 | 0.27±0.35 | 82.0 | 0.0029 |
| HM910 l-dose | 18 | 8 | 8 | 21.0 ±1.6 | 19.2 ±0.9 | 48.6 ±4.9 | 653.0± 560.4 | 13.5±11.0 | 66.5 | 0.0035 | 0.41±0.34 | 72.7 | 0.0063 |

Table 4 and Figure 3 show, Topotecan group (2 mg/kg) had no significant anti-tumor effect on human multiple myeloma NCI-H929 cells in nude mice, whereas HM910 high dose (35 mg/kg), the dose (25 mg/kg) and low-dose group (18 mg/kg) had significant anti-tumor effect on multiple myeloma cells NCI-H929 xenografts in nude mice.

Camptothecin derivatives according to the present invention have significant effects in terms of growth inhibition of bone marrow tumor, and also show beneficial anti-resistance, and thus have advantages over conventional therapeutic agent for treating myeloma.

Example 4: Water-solubility test of compounds of Formula II and Formula IV

Table 5 shows the Water-solubility of compounds of Formula II and Formula IV of the present invention.

Table 5: Water-solubility of compounds of Formula II and Formula IV

| No. of Comp of Formula II | Water-solubility of Comp of Formula IV | Water-solubility of Comp of Formula II |
|---|---|---|
| WQ1001 | <0.01 mg/mL | > 10 mg/mL |
| WQ1102 | <0.01 mg/mL | > 10 mg/mL |
| WQ1103 | <0.01 mg/mL | > 10 mg/mL |
| WQ1104 | <0.01 mg/mL | > 10 mg/mL |
| WQ1105 | <0.01 mg/mL | > 10 mg/mL |
| WQ1106 | <0.01 mg/mL | > 10 mg/mL |
| WQ1107 | <0.01 mg/mL | > 10 mg/mL |
| WQ1108 | <0.01 mg/mL | > 10 mg/mL |
| WQ1109 | <0.01 mg/mL | > 10 mg/mL |
| WQ1110 | <0.01 mg/mL | > 10 mg/mL |
| WQ1111 | <0.01 mg/mL | > 10 mg/mL |
| WQ1112 | <0.01 mg/mL | > 10 mg/mL |
| WQ1113 | <0.01 mg/mL | > 10 mg/mL |
| WQ1114 | <0.01 mg/mL | > 10 mg/mL |
| WQ1115 | <0.01 mg/mL | > 10 mg/mL |
| WQ1116 | <0.01 mg/mL | > 10 mg/mL |
| WQ1117 | <0.01 mg/mL | > 10 mg/mL |
| WQ1118 | <0.01 mg/mL | > 10 mg/mL |
| WQ1119 | <0.01 mg/mL | > 10 mg/mL |
| WQ1120 | <0.01 mg/mL | > 10 mg/mL |
| WQ1121 | <0.01 mg/mL | > 10 mg/mL |
| WQ1122 | <0.01 mg/mL | > 10 mg/mL |
| WQ1123 | <0.01 mg/mL | > 10 mg/mL |
| WQ1124 | <0.01 mg/mL | > 10 mg/mL |
| WQ1125 | <0.01 mg/mL | > 10 mg/mL |
| WQ1126 | <0.01 mg/mL | > 10 mg/mL |

## Claims

**1.** Use of a camptothecin derivative or pharmaceutically acceptable salts thereof in manufacturing a medicament for treatment of multiple myeloma,

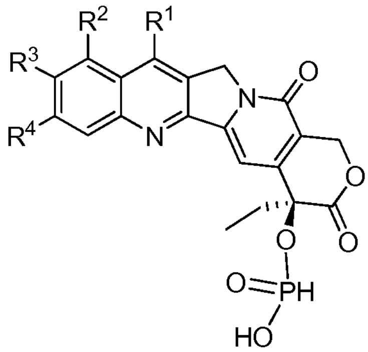

Formula I

wherein

$R^1$ and $R^2$ independently represent hydrogen, hydroxy, nitro, cyano, halo, carboxy, optionally substituted amino, a silyl or siloxyl group containing C1-C6 alkyl group, mono-ring aryloxy, C1-C6 alkanoyl optionally substituted by hydroxy, nitro, cyano, halo or amino, C3-C6 cycloalkyl optionally substituted by hydroxy, nitro, cyano, halo or amino, C1-C6 alkoxy optionally substituted by hydroxy, nitro, cyano, halo or amino, or C1-C6 acyl optionally substituted by hydroxy, nitro, cyano, halo or amino; or $R^1$ and $R^2$ are connected via one to three other atoms forming a heterocycle;

$R^3$ and $R^4$ independently represent hydrogen, hydroxy, nitro, cyano, halo, carboxy, optionally substituted amino, a silyl or siloxyl group containing a C1-C6alkyl group, mono-ring aryloxy, C1-C6 alkanoyl optionally substituted by hydroxy, nitro, cyano, halo or amino, C3-C6 cycloalkyl optionally substituted by hydroxy, nitro, cyano, halo or amino, C1-C6 alkoxy optionally substituted by hydroxy, nitro, cyano, halo or amino, or C1-C6 acyl optionally substituted by hydroxy, nitro, cyano, halo or amino; or $R^3$ and $R^4$ independently represent oxygen and are connected via one or two canbon atoms forming a heterocycle.

**2.** The use of Claim 1, wherein cationic moiety $X^{n+}$ is selected from $K^+$, $Na^+$, $Li^+$, $Mg^{2+}$, $Ca^{2+}$, $Zn^{2+}$, $Fe^{3+}$ or ammonium ion.

**3.** The use of Claim 2, wherein the ammonium ion is derived from one of following bases: $NH_3$, monomethylamine, dimethylamine, trimethylamine, monoethylamine, diethylamine, triethylamine, methylethylamine, dimethylethylamine, diisopropylamine, pyrrolidine, dihydro-isoindol, morpholine, N,N-diallyl amine, 4-methyl piperidine, ethanolamine, 5-bromo dihydro-isoindol, thiomorpholine, cis-2,6-dimethylmorpholine and ethylenediamine.

**4.** The use of Claim 1, wherein the campthothcin derivative is derived from at least one selected from a group consisting of campthothcin, 10,11-ethylenedioxy CPT, 7-ethyl-10,11-ethylenedioxy CPT, 10,11-methylenedioxy CPT, 7-ethyl-10,11-methylenedioxy CPT, 7-chloro-10,11-methylenedioxy CPT, 7-ethyl CPT,10-methyl-7-ethylCPT, 7-ethyl-10-chloro CPT, 7-ethyl-10-bromo CPT, 7-ethyl-10,11-difluoro CPT, 10-methyl-7-ethyl-11-fluoro CPT, 7-ethyl-10,11-dichloroCPT, Gimatecan, Karenitecan, Silatecan, 10-chloro CPT, 10-methylCPT,10-ethyl CPT, 10-n-propyl CPT, 10-n-butyl CPT, 7-methoxyl CPT, 10-methyl-7-methoxyl CPT, 10-ethyl-7-methoxyl CPT, 10-n-propyl-7-methoxyl CPT, 10-n-butyl-7-methoxyl CPT.

**5.** The use of Claim 2, wherein the campthothcin derivative is derived from campthothcin.

**6.** The use of Claim 1, wherein the medicament further compositing other substances having anti-cancer activity selected from one or more of estrogen receptor modulator, androgen receptor modulator, retinoid receptor modulator, alkylating agents, tumor necrosis factor, tubulin inhibitor, topoisomerase inhibitors, anti-proliferative agents, acyltransferase Inhibitors HMG-CoA reductase inhibitor, protease inhibitor, and adrenal cortical hormone.

**7.** The use of claim 6, wherein the other substance having anti-cancer activity is one or more of tamoxifen, raloxifene, idoxifene, finasteride, nilutamide, flutamide, bicalutamide, bexarotene, vitamin A acid, 13-cis- retinoic acid, 9-cis-retinoic acid, melphalan, ifosfamide, carboplatin, ranimustine, fotemustine, oxaliplatin, mitoxantrone, paclitaxel, topotecan, trimetrexate, fludarabine, capecitabine, thalidomide, lenalidomide, pomalidomide, prednisone, dexamethasone or bortezomid.

**8.** The use of claim 6, wherein the medicament is dosage form for oral, injected, mucosal or transdermal administration.

**9.** The use of claim 1, wherein the medicament is tablets, capsules, granules, oral liquid, injection liquid, patches or gels.

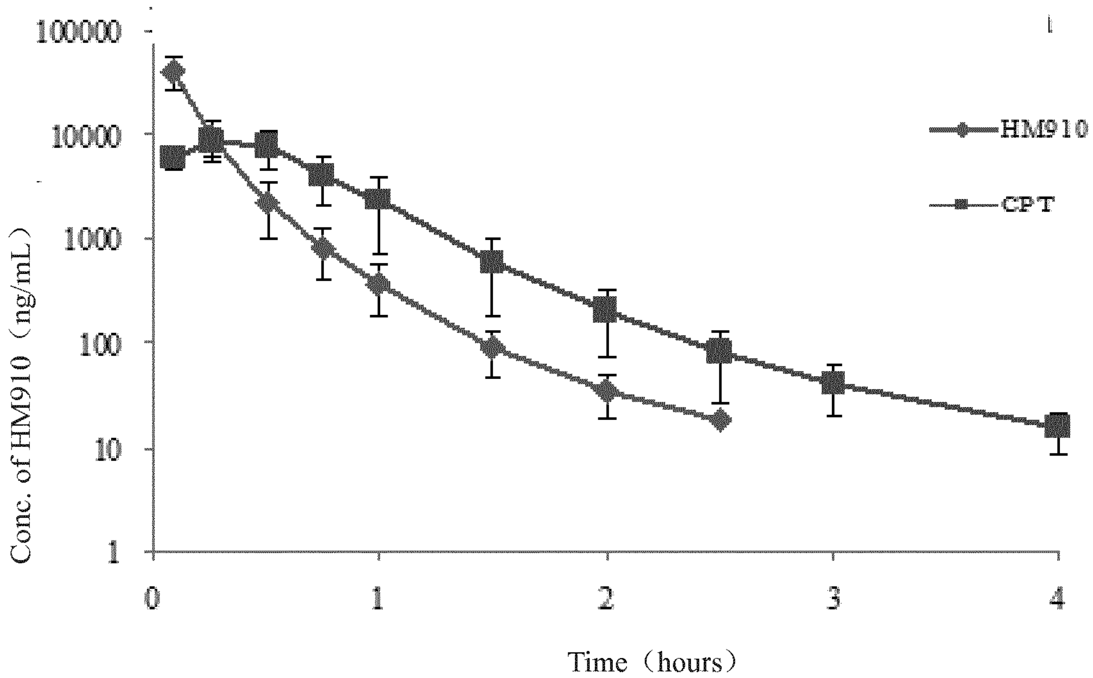
100000
10000
1000
100
10
1
Conc. of HM910（ng/mL）
0
1
2
3
4
Time（hours）
HM910
CPT

FIG. 1

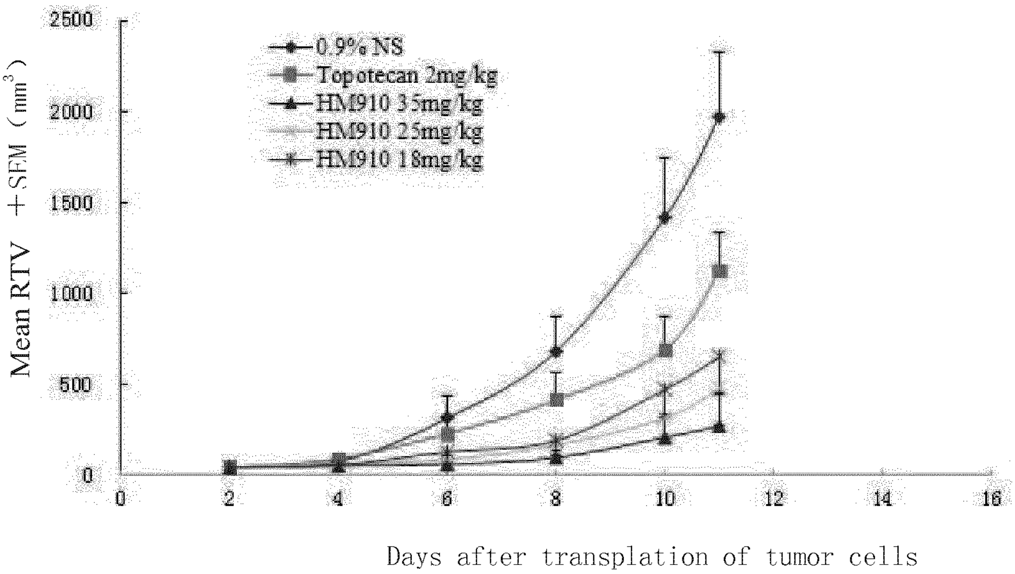
2500
2000
1500
1000
500
0
Mean RTV +SEM（mm³）
0
2
4
6
8
10
12
14
16
0.9% NS
Topotecan 2mg/kg
HM910 35mg/kg
HM910 25mg/kg
HM910 18mg/kg
Days after transplation of tumor cells

FIG. 2

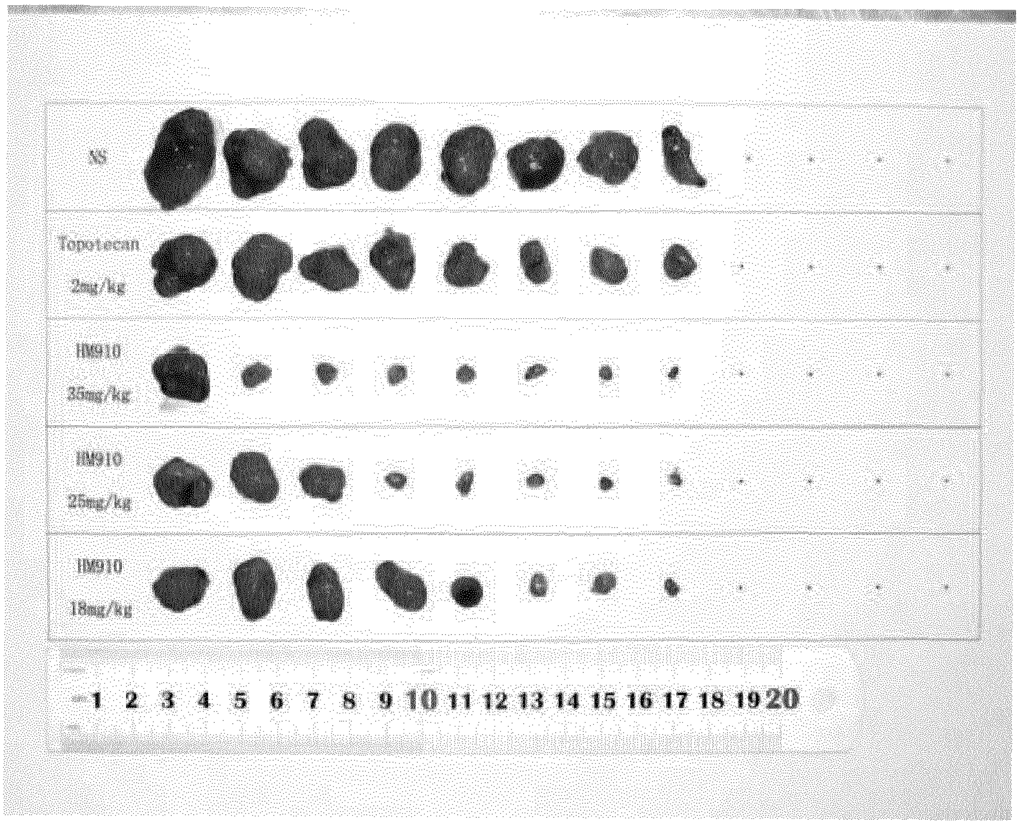
NS
Topotecan
2mg/kg
HM910
35mg/kg
HM910
25mg/kg
HM910
18mg/kg
1 2 3 4 5 6 7 8 9 10 11 12 13 14 15 16 17 18 19 20

FIG. 3

## INTERNATIONAL SEARCH REPORT

International application No.
**PCT/CN2014/000111**

**A. CLASSIFICATION OF SUBJECT MATTER**

A61K 31/675 (2006.01) i; A61P 35/00 (2006.01) i

According to International Patent Classification (IPC) or to both national classification and IPC

**B. FIELDS SEARCHED**

Minimum documentation searched (classification system followed by classification symbols)

A61K, A61P

Documentation searched other than minimum documentation to the extent that such documents are included in the fields searched

Electronic data base consulted during the international search (name of data base and, where practicable, search terms used)

WPI, EPODOC, CPRS (CN), CNKI (CN), REGISTRY, CAPLUS: camptothecin, gimatecan, karenitecan, silatecan, multiple myeloma, the structure of formula I

**C. DOCUMENTS CONSIDERED TO BE RELEVANT**

| Category* | Citation of document, with indication, where appropriate, of the relevant passages | Relevant to claim No. |
|---|---|---|
| A | CN 102850400 A (ZHOU, Wenqiang), 02 January 2013 (02.01.2013), see the whole document, particularly claim 10 | 1-9 |
| A | XU, Shufen et al., "A Preliminary Report of the Treatment of Multiple Myeloma with VHEP Regimen", MEDICAL JOURNAL OF NATIONAL DEFENDING FORCES IN NORTHWEST CHINA, vol. 30, no. 4, 31 August 2009 (31.08.2009), pages 244-246, see abstract | 1-9 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 22 August 2014 (22.08.2014) | **03 September 2014 (03.09.2014)** |

Name and mailing address of the ISA/CN:
State Intellectual Property Office of the P. R. China
No. 6, Xitucheng Road, Jimenqiao
Haidian District, Beijing 100088, China
Facsimile No.: (86-10) 62019451

Authorized officer
**HUANG, Yijie**
Telephone No.: (86-10) **62411202**

Form PCT/ISA/210 (second sheet) (July 2009)

**INTERNATIONAL SEARCH REPORT**
Information on patent family members

International application No.
**PCT/CN2014/000111**

| Patent Documents referred in the Report | Publication Date | Patent Family | Publication Date |
|---|---|---|---|
| CN 102850400 A | 02 January 2013 | WO 2013000269 A1 | 03 January 2013 |
| | | AU 2012276175 A1 | 09 January 2014 |
| | | CA 2838875 A1 | 03 January 2013 |
| | | KR 20140020333 A | 18 February 2014 |
| | | US 2014107342 A | 17 April 2014 |
| | | EP 2727927 A1 | 07 May 2014 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 102850400 A


### Non-patent literature cited in the description


- *The New England Journal of Medicine,* 2011, vol. 364, 1046-60
- *Clinical Lymphoma & Myeloma,* 2009, vol. 9 (4), 278-288
- *Blood,* 2008, vol. 111, 2521-2526
- *J Clin Oncol,* 1998, vol. 16, 589-592
- *Leukemia & Lymphoma,* 2004, vol. 45 (4), 755-759
- *Bone Marrow Transplantation,* 2011, vol. 46, 510-515
- *Nature Review/Cancer,* October 2006, vol. 6, 789-802
- *Cancer Res,* 1999, vol. 59, 5938-5946
- *Blood,* 2006, vol. 108 (12), 3881-3889
- *Biochem Pharmacol.,* 2012, vol. 83 (8), 1084-1103